# NEW EUROPEAN PATENT SPECIFICATION

(11) **EP 0 336 731 B2**
(45) Date of publication and mention of the opposition decision: **07.01.2004**
(45) Mention of the grant of the patent: 11.05.1994
(21) Application number: 89303351.4
(22) Date of filing: 05.04.1989
(51) Int. Cl.: C12Q 1/68

(54) **Method of amplifying and detecting nucleic acid sequences**
Verfahren zur Vermehrung und zum Nachweis von Nukleinsäuresequenzen
Procédé pour l'amplification et détection des séquences d'acides nucléiques

(30) Priority: 06.04.1988 US 178377
(43) Date of publication of application: 11.10.1989
(73) Proprietor: CITY OF HOPE, Duarte California 91010-0269 (US)
(72) Inventor: Wallace, Bruce R., South Pasadena, CA 91030 (US)
(74) Representative: Marchant, James Ian

(56) References cited:
- EP-A- 0 123 513
- EP-A- 0 130 515
- EP-A- 0 185 494
- EP-A- 0 201 184
- EP-A- 0 246 864
- EP-A- 0 320 308
- WO-A-93/20227
- US-A- 683 202
- US-A- 4 358 535
- US-A- 4 683 195
- US-A- 5 582 989
- US-A- 5 686 272
- NATURE, vol. 324, 13 November 1986, London (GB); R.K. SAIKI et al., pp. 163-166#
- GENOMICS, vol. 2, no. 4, 1988, San Diego, CA (US); M.H. SKOLNICK et al., pp. 273-279#
- GENOMICS, vol. 4, no. 4, 1989, San Diego, CA (US); D.Y. WU et al., pp. 560-569#
- GENE, vol. 76, no. 2, February 1989, Amsterdam (NL); D.Y. WU et al., pp. 245-254#
- SCIENCE, vol. 241, 26 August 1988, Washington, DC (US); U. LANDEGREN et al., pp. 1077-1080#
- Molecular cloning, a Lab. Manual 2nd Edn. Cold Spring Harbor Press. 1989.
- Primer orientation.(a sequence taken from "Molecular cloning" Lab manual)
- Gene cloning. D Glover. p.24. Pub : Chapman and Hall ltd. 1984
- Boehringer Mannheim catalogue. Biochemicals for Molecular Biology. End Labelling kit. p125-126
- Gene. Vol26, p 101-106. Norrander et al. 1983
- DNA. Vol 6,No 5, p.497-504. Ebe et al. 1987
- Declaration of Dr K Backman
- Biotechniques, vol 17, No 1, p 82-85. He et al. 1994
- PNAS vol 80, p 5852-5856, Zimmerman et al. 1983
- J Mol Biol, vol 45, p 513-531, Cozzarelli et al. 1969

## Description

This invention relates to a method of amplifying nucleic acid sequences.

Methods are known for enzymatically joining portions of nucleic acids together. Weiss, B., et al., J. Biol. Chem. 245(17):4543 (1968) describe joining two segments of an interrupted strand in a DNA duplex using T4-polynucleotide (DNA) ligase. Sgaramella, V., et al., Proc. Natl. Acad. Sci. U.S.A. 67(3):1468 (1970) teach use of T4-polynucleotide (DNA) ligase to join blunt ends of DNA duplexes. Nath, K., et al., J. Biol. Chem. 249(12):3680 (1974) describe using DNA ligase to attach polyribonucleotides to polydeoxyribonucleotides. Higgins, N.P., et al., Methods in Enzymology 68:50 (1979) provides a review of DNA-joining enzymes. Harrison, B., et al., Nucleic Acids Research 12:8235 (1984) teach an enhanced ligation of oligonucleotides and polynucleotides by T4 RNA ligase in polymer solutions.

With respect to amplification, Saiki, R.K., et al., Science 230:1350 (1985) describes enzymatic amplification of β-globin genomic sequences, termed the polymerase chain reaction, by providing two oligonucleotide primers flanking the region to be amplified, annealing the primers to strands of denatured genomic DNA and extending them with the Klenow fragment of E. coli DNA polymerase 1 and deoxyribonucleosidetriphosphates. U.S. Patent 4,683,202 to Mullis further describes the polymerase chain reaction method.

U.S. Patent 4,683,194 to Saiki, et al., describes a method of detecting a specific polymorphic restriction site in a nucleic acid sequence by hybridizing the sequence to an oligonucleotide probe complementary to the sequence and spanning the restriction site which is labeled at the end of the probe nearer to the restriction site. The nucleic acid sequence and probe hybridized thereto are then digested with a restriction endonuclease, labeled and unlabeled oligomer fragments are separated, and labeled oligonucleotides are detected.

U.S. Patent 4,683,195 to Mullis et al. describes a process for amplifying nucleic acid sequences using primers and polymerization agents and a process for detecting the presence or absence of a specific nucleic acid sequence. Separate strands of a nucleic acid sample are treated with an excess of two different oligonucleotide primers, and primers are extended to form complementary extension products which act as templates for synthesizing the desired nucleic acid sequence. The amplified sequence is then detected by adding a labeled probe capable of hybridizing to the sequence being detected.

EP 0 246 864 discloses a split probe hybrid assay wherein a target base sequence lies at the terminal end of a first probe and said terminal end is contiguous with a second probe which may then be linked, by means of a ligase enzyme, to the first probe. The signal thus produced is then amplified, in contrast to the oresent invention wherein the target base sequence itself is amplified.

The present invention is useful in diagnostic applications because it can be used first, as a means for amplifying given nucleic acid sequences from nucleic acid templates so that the template sequences can be more readily detected.

In order to amplify a given nucleic acid sequence comprising a target sequence a single-stranded template is generated comprising the target sequence and at least one pair of oligonucleotides is hybridised to contiguous sequences of the template such that the oligonucleotides are juxtaposed for ligation on the template. The juxtaposed oligonucleotides are ligated to produce a double-stranded ligation product and the double-stranded ligation product is denatured to produce an initial template strand and an oligonucleotide ligation product strand. These steps are repeated such that both the initial template strand and the oligonucleotide ligation strand act as templates for a further ligation reactions.

While there exists the possibility of ligation under mismatched conditions in which the base complementary to the end base of one of the oligonucleotides at the ligation junction is not present in a given template, it has been found, according to the present invention, that such ligation can be suppressed so that a base mismatch on either side of the ligation junction reduces the efficiency of ligation between the two oligonucleotide strands and serves as the theoretical basis to distinguish single base differences on a nucleic acid template. The methods of the present invention can therefore also be used as a diagnostic technique to detect single base or base pair mutations in nucleic acid sequences. This further aspect of the present invention is therefore useful to diagnose genetic diseases, such as sickle cell anemia, hemophilia, and color blindness which result from a single point mutation on a critical structure gene.

### SUMMARY OF THE INVENTION

According to a first aspect, the present invention provides a method for amplifying a nucleic acid comprising a target sequence comprising the steps of:
(a) generating a single-stranded template comprising the target sequence;
(b) hybridising a pair of oligonucleotides to contiguous sequences of the template, said contiguous sequences comprising the target sequence, such that the oligonucleotides are juxtaposed for ligation on the template;
(c) ligating the juxtaposed oligonucleotides to produce a double-stranded ligation product;
(d) denaturing the double-stranded ligation product to produce an initial template strand and an oligonucleotide ligation product strand; and
(e) repeating steps (b) to (d) such that both the initial template strand and the oligonucleotide ligation strand act as templates for a further ligation reaction;
wherein steps (b) to (e) are conducted in a reaction mixture which contains at least 200 mM NaCl.

According to a second aspect, the present invention provides a method for amplifying a nucleic acid comprising a target sequence, said method comprising the steps of:
(a) generating a single-stranded template comprising the target sequence;
(b) amplifying said template by the polymerase chain reaction (PCR) to produce a PCR amplification product comprising multiple copies of said template comprising the target sequence;
(c) hybridising pairs of oligonucleotides to contiguous sequences of said copies of said template, said contiguous sequences comprising the target sequence, such that the oligonucleotides are juxtaposed for ligation on said template copies;
(d) ligating the juxtaposed oligonucleotides to produce double-stranded products;
(e) denaturing the double-stranded products to produce a plurality of initial template strands and a plurality of oligonucleotide product strands; and
(f) repeating steps (c) to (e) such that both the initial template strands and the oligonucleotide ligation strands act as templates for a further ligation reaction.

According to a third aspect, the present invention provides a method for simultaneously amplifying a plurality of nucleic acid sequences present in one or more nucleic acid molecules each comprising a target sequence, said method comprising the steps of:
(a) generating as appropriate a single-stranded template comprising the target sequences or a plurality of single-stranded templates each comprising a target sequence;
(b) hybridising a plurality of pairs of oligonucleotides to contiguous sequences of said template(s), said contiguous sequences comprising the target sequences, such that each of said oligonucleotide pairs is juxtaposed for ligation on a template;
(c) ligating the so juxtaposed oligonucleotide pairs to produce double-stranded ligation product;
(d) denaturing double-stranded ligation product to produce initial template strands and oligonucleotide ligation product strands, and
(e) repeating steps (b) to (d) such that both initial template strands and oligonucleotide ligation product strands act as templates for further ligation reactions.

According to a fourth aspect, the present invention provides a method for the diagnosis of a genetic disease comprising analysing the genotype of a patient by a method which comprises amplifying a nucleic acid comprising a target sequence, said amplification method comprising the steps of :
(a) generating a single-stranded template comprising the target sequence;
(b) hybridising a pair of oligonucleotides to contiguous sequences of the template, said contiguous sequences comprising the target sequence, such that the oligonucleotides are juxtaposed for ligation on the template;
(c) ligating the juxtaposed oligonucleotides to produce a double-stranded ligation product;
(d) denaturing the double-stranded ligation product to produce an initial template strand and an oligonucleotide ligation product strand; and
(e) repeating steps (b) to (d) such that both the initial template strand and the oligonucleotide ligation product strand act as templates for a further ligation reaction.

According to a fifth aspect, the present invention provides a method for amplifying a nucleic acid comprising a target sequence from genomic DNA, said amplification method comprising the steps of:
(a) generating a single-stranded template comprising the target sequence;
(b) hybridising a pair of oligonucleotides to contiguous sequences of the template, said contiguous sequences comprising the target sequence, such that the oligonucleotides are juxtaposed for ligation on the template;
(c) ligating the juxtaposed oligonucleotides to produce a double-stranded ligation product;
(d) denaturing the double-stranded ligation product to produce an initial template strand and an oligonucleotide ligation product strand; and
(e) repeating steps (b) to (d) such that both the initial template strand and the oligonucleotide ligation product strand act as templates for a further ligation reaction.

According to a sixth aspect, the present invention provides a method for amplifying a nucleic acid comprising a target sequence comprising the steps of:
(a) generating a single-stranded template comprising the target sequence;
(b) hybridising a pair of oligonucleotides to contiguous sequences of the template, said contiguous sequences comprising the target sequence, such that the oligonucleotides are juxtaposed for ligation on the template;
(c) ligating the juxtaposed oligonucleotides to produce a double-stranded ligation product;
(d) denaturing the double-stranded ligation product to produce an initial template strand and an oligonucleotide ligation product strand; and
(e) repeating steps (b) to (d) such that both the initial template strand and the oligonucleotide ligation strand act as templates for a further ligation reaction;
wherein the template nucleic acid is from bacteria.

According to a seventh aspect, the present invention provides a method for amplifying a nucleic acid comprising a target sequence comprising the steps of:
(a) generating a single-stranded template comprising the target sequence;
(b) hybridising a pair of oligonucleotides to contiguous sequences of the template, said contiguous sequences comprising the target sequence, such that the oligonucleotides are juxtaposed for ligation on the template;
(c) ligating the juxtaposed oligonucleotides to produce a double-stranded ligation product;
(d) denaturing the double-stranded ligation product to produce an initial template strand and an oligonucleotide ligation product strand; and
(e) repeating steps (b) to (d) such that both the initial template strand and the oligonucleotide ligation strand act as templates for a further ligation reaction;
wherein the template nucleic acid is from yeast.

According to an eighth aspect, the present invention provides a method for amplifying a nucleic acid comprising a target sequence comprising the steps of:
(a) generating a single-stranded template comprising the target sequence;
(b) hybridising a pair of oligonucleotides to contiguous sequences of the template, said contiguous sequences comprising the target sequence, such that the oligonucleotides are juxtaposed for ligation on the template;
(c) ligating the juxtaposed oligonucleotides to produce a double-stranded ligation product;
(d) denaturing the double-stranded ligation product to produce an initial template strand and an oligonucleotide ligation product strand; and
(e) repeating steps (b) to (d) such that both the initial template strand and the oligonucleotide ligation strand act as templates for a further ligation reaction;
wherein the template nucleic acid is from a virus.

Other advantages and features of the invention will be apparent from the following description of the preferred embodiments thereof, and from the claims.

### DESCRIPTION OF THE DRAWINGS

Fig. 1 illustrates a 35 base pair length template of the normal human (β^{a}) beta globin gene in the region of the allelic base pair DNA polymorphism associated with sickle cell anemia and four oligodeoxyribonucleotide substrate strands complementary to the normal beta globin allele of the gene to be used for ligation amplification.
Fig. 2 illustrates a 35 base pair length template of the human sickle cell (β^{s}) beta globin gene in the region of the allelic base pair DNA polymorphism which is associated with sickle cell anemia and four oligodeoxyribonucleotide substrate strands complementary to the sickle cell allele of the gene to be used for ligation amplification.
Fig. 3 is a schematic diagram of template directed exponential ligation amplification.
Fig. 4 is a photograph of an autoradiogram of product of linear amplification ligation of β^{s} flanking oligonucleotide substrates on sickle cell (lanes c and g) and normal beta globin (lanes d and h) oligonucleotide templates.
Fig. 5A is a photograph of autoradiograms of reaction products from six rounds of exponential amplification ligation of flanking oligonucleotide substrates designed to be complementary to the sickle cell gene sequence using sickle cell (Hβ23S') and normal (Hβ23A') oligonucleotide template.
Fig. 5B shows a logarthmic plot of fold of amplification versus number of exponential amplification ligation rounds performed.
Fig. 6 is a photograph of an autoradiogram containing ligation amplification product from 10-14 exponential rounds of ligation amplification using templates from plasmids containing normal (lanes g-i) and sickle cell (lanes d-f) beta globin gene using flanking oligonucleotide substrates designed to be complementary to the sickle cell beta globin gene sequence (Fig. 2) and normal beta globin gene sequence (Fig. 1).
Figs. 7A and 7B are photographs of autoradiograms resulting from amplification of human genomic DNA using β thalassemia (lanes a, b), sickle cell (lanes c, d), and normal β globin (lanes e, f) DNA template. Fig. 7A resulted from ligating β^{s} flanking oligonucleotides; Fig. 7B resulted from ligating β^{a} flanking oligonucleotides.
Fig. 8 is a photograph of an autoradiogram containing linear ligation amplification product using a pair of β^{s} flanking oligonucleotides and human genomic DNA template from β-thalassemia (lane a), sickle cell (lane b), and normal β globin (lane c). Lanes d-f are controls.
Fig. 9A is a photograph of an ethidium bromide stained agarose gel containing PCR amplified 294 base pair fragments of alleles at the human beta globin gene.
Figs. 9B(a) and 9B(b) are photographs of autoradiograms of PCR products from the Fig. 9A gel transferred to nylon membranes and hybridized with radiolabeled Hβ19S (panel a) and radiolabeled Hβ19A (panel b) probes.
Fig. 9C is a photograph of an autoradiogram of ligation amplification products using PCR enriched sequences as templates.

### DETAILED DESCRIPTION OF THE INVENTION

The method of the present invention can be used both to amplify any particular nucleic acid sequence template and distinguish and detect single base differences on nucleic acid templates. The present invention is therefore useful to diagnose various genetic disorders that result from point mutations such as sickle cell anemia. In other diagnostic applications, signal amplification is necessary in order to detect the limited number of DNA template copies in genomic DNA. For example, there are only about one million copies of a single gene in 10 ug of DNA. Further, depending on the particular diagnostic procedure performed, only a limited amount of DNA might be available.

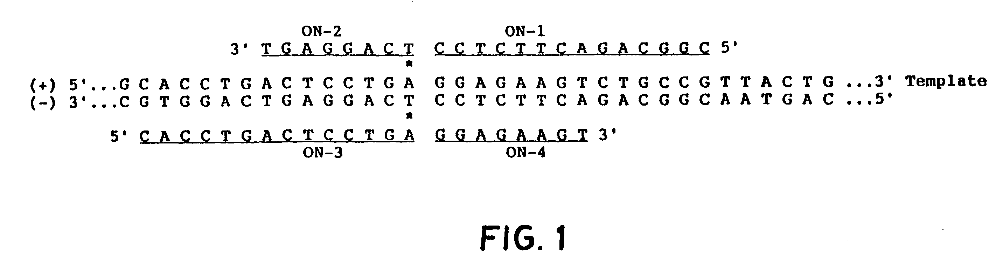

According to the methods of the present invention, short oligonucleotide strands are synthesized complementary to a nucleic acid template. These relatively short oligonucleotides are generally denoted ON-1, ON-2, ON-3, and ON-4 as illustrated in Fig. 1. The oligonucleotides may be synthesized according to methods well known in the art.

The lengths of the oligonucleotides must be sufficiently long to contain enough of a unique base pattern to cause the oligonucleotides to hybridize or anneal to only the portions of the nucleic acid template which constitutes the target sequence of interest. The exact lengths of the oligonucleotides depends on various factors, including the temperature of the reaction, pH conditions, and complexity of the target sequence. In general, oligonucleotides comprising about 4 to about 100 nucleotides form sufficiently stable hybrid complexes at higher reaction temperatures. In most instances, oligonucleotides used in accordance with the present invention preferably comprise about 8 to about 20 nucleotides.

The methods of the present invention may be carried out with nucleic acid template which is either single or double stranded. If the nucleic acid template comprises two strands, it is necessary to first separate the strands prior to commencing ligation amplification of the present invention. One method of separation of strands of nucleic acid involves heating the nucleic acid until it is completely denatured. Such denaturation is usually carried out at temperatures ranging from about 80° to 105°C for times ranging from about 1 to 10 minutes. Separation of nucleic acid strands may also be induced by enzymes such as the helicase enzymes and Rec A. It is also possible to remove one of two strands by digesting with appropriate restriction endonuclease followed by Exonuclease III (Exo III) or Lambda Exonuclease as described by Wallace, R.B., et al. Science 209:1396 (1980).

The specific nucleic acid sequence to be amplified may be only a fraction of a larger molecule or it can be a discrete molecule. The sequence to be amplified can also be a part of a larger mixture of molecules or fractions thereof. The starting nucleic acid may also contain more than one desired sequence containing target bases which may be the same or different, such that all such sequences can be simultaneously amplified. Samples comprising more than one nucleic acid with more than one desired target sequence can also be amplified by the methods of the present invention.

Any source of template nucleic acid can be employed to practice the present invention as long as it contains or is suspected to contain the specific nucleic acid sequence desired. For example, plasmids, natural DNA or RNA, from bacteria, yeast, viruses, plants or animals, or cloned DNA or RNA can be used. Genomic DNA or total RNA may also be extracted from human blood and tissue by a variety of techniques. Synthetic oligodeoxyribonucleotides may also be used. When RNA is used as an initial template, it can be first copied into a DNA molecule by an enzyme such as reverse transcriptase.

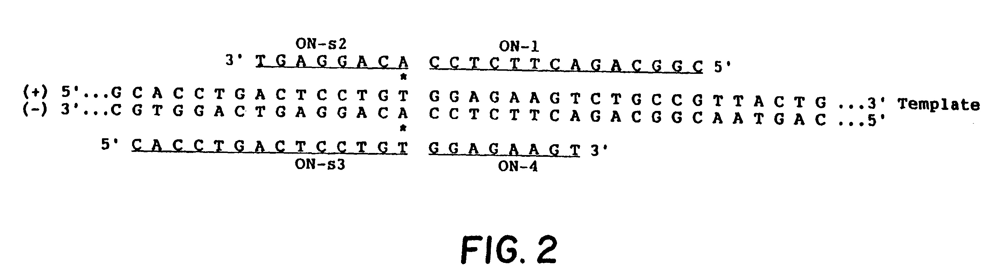

As seen in Fig. 1, the template is the β^{a} normal beta globin allele with target base pair *A-T*; in Fig. 2, the template is the β^{s} sickle cell allele with target base pair *T-A*. It must be understood that the particular templates and flanking oligonucleotides shown in Figs. 1 and 2 only illustrate the present invention and in no way limit the invention to the detection or amplification of alleles of the human beta globin gene.

As shown in Fig. 1, the 5'-3' (upper) strand of the template is denoted (+); the 3'-5' (lower) strand is denoted (-). Oligonucleotides ON-2 and ON-4 are 8-nucleotide long oligonucleotides and ON-1 and ON-3 are 14-nucleotide long oligonucleotides. The method of the present invention requires a symmetry similar to that shown in Fig. 1, namely, the 3' oligonucleotides, ON-2 and ON-4, are of the same length and the 5' oligonucleotides, ON-1 and ON-3, are the same length. Either the two 3' or two 5' oligonucleotides can be the longer length, or all four oligonucleotides can be the same length. The 5' ends of the 3' oligonucleotides (ON-2 and ON-4) must, however, be phosphorylated and those ends can also be optionally labeled with, for example, radioactive phosphorous. The 5' oligonucleotides (ON-1 and ON-3) are not usually phosphorylated to prevent self ligation and template-independent ligation.

The methods of the present invention are not, however, limited to the above-described instances involving single base or single base pair differences. The oligonucleotide complementary region can thus contain more than one base or base pair difference which enables the methods of this invention to be used for discrimination and detection of sequences containing multiple base differences.

It is necessary that in the case of double-stranded nucleic acid templates the two sets of oligonucleotides define a blunt end as seen in Fig. 1, as opposed to a staggered end.

The pairs of oligonucleotides are complementary to a nucleic acid sequence harboring the position of possible mutation on either strand. Further, with double-stranded templates, one of the terminal bases of one of the two 3' oligonucleotides and one of the terminal bases of one of the two 5' oligonucleotides to be ligated must be complementary to a base pair in the target sequence. Thus, as shown in Fig. 1, the base at the 5' end of one of the 3' flanking oligonucleotides (base T, ON-2) is complementary to the base A of the (+) template strand, and the nucleotide at the 3' end of one of the 5' flanking oligonucleotides (base A, ON-3) is complementary to the base T of the (-) template strand.

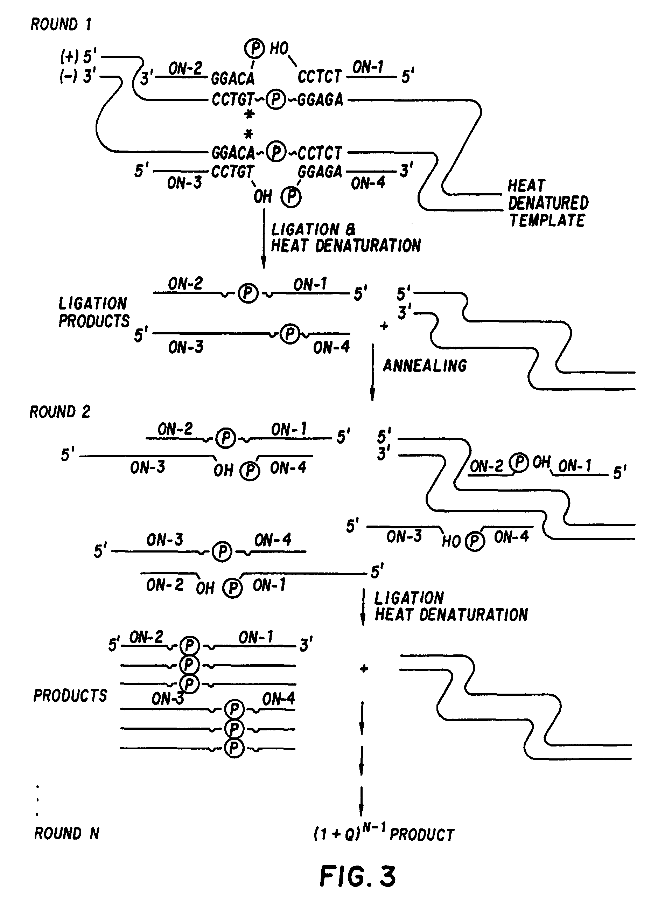

As seen in Fig. 1, 3' oligonucleotide ON-2 is synthesized so that it is complementary to the ( + ) or upper strand of the template and its 5' end contains base T which is complementary to base A of the (+) strand, and the remainder of the oligonucleotide extends outwardly from the 5' end base. 5' oligonucleotide ON-1 is synthesized so that it is complementary to the (+) or upper strand of the template and is oriented such that the base at its 3' end C, is complementary to the base of the (+) strand on the 3' side of the base A on the template. The remainder of the oligonucleotide extends outwardly in the 5' direction.

5' oligonucleotide ON-3 is complementary to the (-) or lower strand of the template such that base A at its 3' end is complementary to the base T of the (-) strand and the remainder of ON-3 extends outwardly from that base. 3' oligonucleotide ON-4 is complementary to the (-) or lower strand of the template and is oriented such that base G at the 5' end of ON-4 is complementary to the base of the (-) strand that is on the 5' side of the base T. The remainder of ON-4 extends outwardly in the 3' direction. Thus, the bases on the 5'end of ON-2 and 3' end of ON-3 are complementary to the A-T base pair shown in the target sequence.

When double-stranded nucleic acid template is used, symmetry requires that ON-2 be complementary to ON-3, except for differences in length. Likewise ON-1 is complementary to ON-4, except for differences in length. As seen in Fig. 1, ON-1 is complementary to ON-4. Therefore in cases where double-stranded nucleic acid template is to be used, the oligonucleotides can be double-stranded, for example: 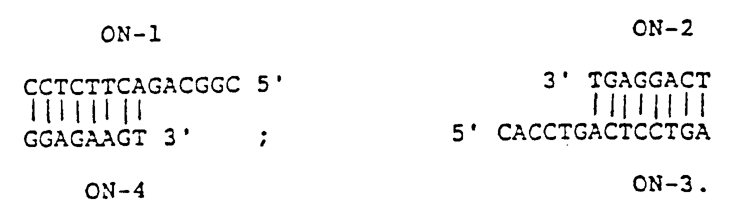 Such double-stranded oligonucleotides must be treated as described above to separate the strands prior to any amplification ligation reaction.

At appropriate temperature and assay conditions polynucleotide ligase ligates ON-1 and ON-2, and/or ON-3 and ON-4 to form significant amounts of longer ligated products only if the correct complementary target bases are present at the site of the template corresponding to the ligation junction. Any suitable polynucleotide ligase can be used in the practice of this invention, for example, bacteriophage T4 induced DNA ligase, and E. coli DNA ligase.

It has been found that inclusion of at least 200 mM NaCl in amplification reaction mixtures almost completely supppresses mismatch ligation. Therefore, with reference to Fig. 1, if the ligation junction site of the template DNA contains a nucleotide which is not complementary to 5' end of ON-2 or the 3' end of ON-3, the mismatch of nucleotides prevents any significant ligation which would have caused formation of a 22 base long product. The absence of such ligation product thereby indicates that the bases of interest are not present on the particular template. For example, if the template in Fig. 1 were the sickle cell (β^{s}) gene in which bases A and T are interchanged at the ligation junction site, there would be no ligation of either ON-2/ON-1 or ON-3/ON-4 as depicted in Figure 1. An expected 22 nucleotide amplification ligation product would thus not be formed.

With respect to the specific oligonucleotides shown in Fig. 2, the two oligonucleotides in each pair anneal to the beta globin template at adjacent positions such that the 3' end of one oligonucleotide (ON-1 or ON-s3) and the 5' end of the other (ON-s2 or ON-4) form a ligatable junction. The nucleotide T or A on the template pairs with either the 3' end nucleotide or the 5' end nucleotide of the substrate depending on which strand, (+) or (-), serves as the template for ligation.

The success or failure of a given ligation amplification procedure can be detected by labeling the oligonucleotides with radioactive or fluorescent labels. For example, ³²P, ³⁵S or any other suitable radionuclide may be used as well as other suitable compounds such as fluorescein or rhodamine or their derivatives. Ligation amplification product can also be ascertained via any subsequent manipulation that allows distinction between longer ligation product and the shorter precursors such as gel electrophoresis chromatography or hybridization. For example, polyacrylamide gel electrophoresis can be used to separate the ligated product from the flanking oligonucleotide reactants according to their sizes. Some of these techniques will be fully described in the examples below.

According to the method of the present invention, the herein described ligation amplifications can be accomplished in a linear or exponential manner as illustrated in the following examples. The steps for exponential ligation-amplification are shown in Fig. 3. The DNA sequences of synthetic oligonucleotide substrates and templates used in the examples are given below in Table 1.

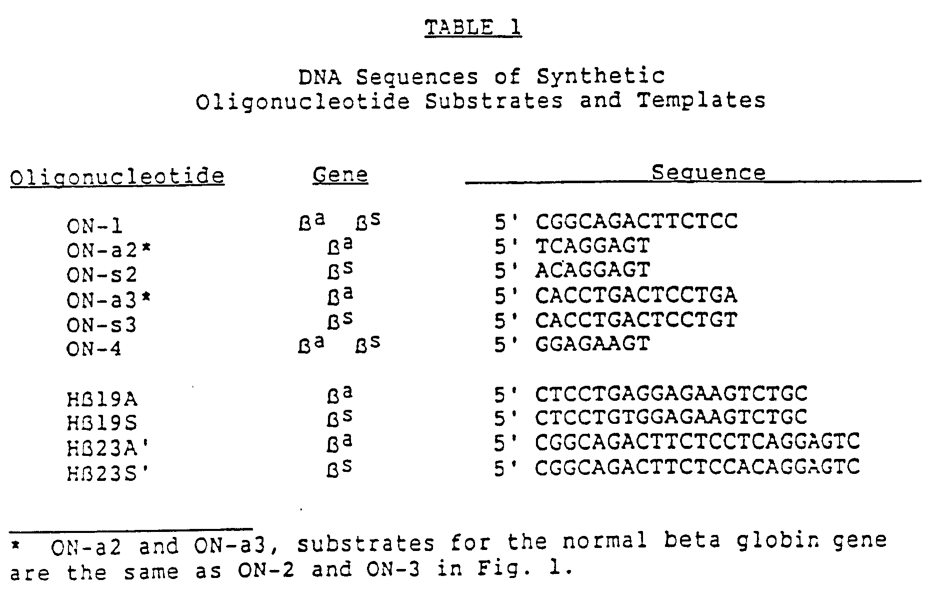

Hβ19S and Hβ19A are unique 19 nucleotide long oligonucleotides that are complementary to the noncoding strand of the beta globin gene and serve as oligonucleotide templates for ligation of ON-1/ON-2 pairs. Hβ23S' and Hβ23A', 23 nucleotides long, are complementary to the coding strand of the beta globin gene. These two nucleotides serve as templates for ligation of ON-3/ON-4 pairs.

While the ON-1/ON-s2 and ON-1/ON-a2 pairs both hybridize to the coding (+) strand, ON-52 and ON-a2 differ by a single nucleotide at their 5' ends, with ON-s2 specifying for the sickle cell allele. Similarly, the ON-s3/ON-4 and ON-a3/ON-4 pairs hybridize to the noncoding strand with ON-s3 and ON-a3 differing by a single nucleotide at their 3' ends.

For the detection of either the β^{a} (normal) or β^{s} (sickle cell) allele, a set comprising at least one of the pairs of oligonucleotide substrates ON-1/ON-a2 and ON-a3/ON-4 are used for the normal beta globin sequence and at least one of the pairs ON-1/ON-s2 and ON-s3/ON-4 are used for detecting the sickle cell sequence.

In the below-described ligation amplification reactions, template dependent ligation of either the ON-1/ON-2 or ON-3/ON-4 pairs forms a 22 base product with a sequence complementary to the other substrate pairs. For example, ON-1-ON-a2 product would be complementary to ON-a3/ON-4 substrates and ON-s3-ON-4 product would be complementary to ON-1/ON-s2 substrates. According to one aspect of the present invention, the ligation products can then serve as template for the ligation of the other pair of oligonucleotide substrates thereby producing more templates for further ligation. If the initial ligation step does not take place as a result of base pair mismatch, subsequent amplification steps will not give rise to a detectable ligation signal.

Once the 22 nucleotide long ligation product is formed, it stays hybridized to its template. Denaturation separates the duplex to yield two new templates. If denaturation is accomplished by boiling, the ligase in the reaction mixture is inactivated. Fresh enzyme must therefore be added to initiate the next round of amplification.

### Example 1

All oligodeoxyribonucleotide ligation reactions on oligonucleotide templates were carried out, unless otherwise indicated, in 50 mM Tris HCl (pH 7.6), 10 mM MgCl₂, 1 mM DTT (dithiothreitol), 1 mM ATP, 200 mM NaCl and 5% PEG (polyethyleneglycol) with 5-10 µm of each substrate oligonucleotide mixed with a small amount of radioactively labeled 3' oligonucleotide substrate (100,000-300,000 cpm) and the indicated amount of template.

Plasmid and genomic DNA template reactions are initiated following 5-10 minutes of boiling, cooling on ice and the addition of ligase (1 U; BRL). Unless otherwise indicated, all ligation reactions are withdrawn for electrophoresis and optionally treated with calf intestinal alkaline phosphatase (1-5 U) for 1-2 hours.

Linear amplification ligation of nucleic acid sequences is carried out by repeatedly using the original nucleic acid template (oligo, plasmid or genomic DNA) in each round of amplification. Only one oligonucleotide set complementary to the appropriate oligonucleotide template to one strand of duplex plasmid or genomic DNA template is used. For example, either the ON-1 and ON-2, or ON-3 and ON-4 set is used in the amplification rounds but not both.

To linearly amplify a sequence of the 5' strand of the human sickle cell (β^{s}) β-globin gene as shown in Fig. 2, the above reaction mixture further containing 1 mM ON-1, 1 mM 5' phosphorylated ON-a2, and 0.1 pmoles of β^{s} (Hβ19S) template was prepared. If double-stranded template is used, it must be denatured by boiling the reaction mixture at about 90-100° C, for 10 minutes.

Each round of ligation is initiated by adding 1 unit of ligase to the reaction mixture on ice. The mixture is incubated for about 30 minutes at 30°C, or for a longer time if template concentration is low.

The first round, and all subsequent rounds, is terminated by quickly heating the reaction mixture to 100°C for 5 minutes to inactivate the ligase and dissociate the ligated product strand (ON-1⁻ON-2) from the template. This frees the template for subsequent amplification rounds. Before the next round, the reaction mixture is centrifuged for 10 seconds and immediately cooled to about 0°C. At this point, there is approximately one ON-1-ON-2 ligation product present for each copy of the template.

Another unit of enzyme is added to the reaction mixture to initiate a second round of ligation. Because a molar excess of oligonucleotides ON-1 and ON-2 are added to the initial reaction mixture, more oligonucleotide substrated are not added. The reaction mixture is then incubated as in the first round, boiled to dissociate template from the ligation product, and cooled. At this point, there are approximately 2 copies of ON-1-ON-2 ligation product present for each copy of template.

Fresh enzyme is added to initiate another round. The steps of incubating, boiling to dissociate template from product, cooling, and adding fresh enzyme are repeated for as many rounds as necessary to produce a detectable signal.

Detection of the ON-1⁻ON-2 ligation product can be accomplished in several ways. ON-1 and/or ON-2 can be labeled with radioactive or nonradioactive labels and separated from the reactants by gel electrophoresis, such that ligation product is determined by detecting the presence of label in a position in the gel corresponding to the expected product size.

ON-a2, ON-s2, and ON-4 are phosphorylated at their 5' ends with either radioactive (γ [³²P] ATP 6000 Ci/mmol; New England Nuclear) or nonradioactive ATP via a kinase reaction. The unlabeled 5' phosphorylated substrates are used following incubation in a boiling water bath for 20 minutes to inactive kinase. Internal radioactively phosphorylated oligonucleotide products are separated from unreacted ATP and other reaction products with anion exchange chromatography (DE-52 cellulose-Whatman®). The radioactive phosphorylation reaction yields products with approximately 10⁸-10⁹ cpm/pmol specific activity.

Detection and quantitation of ligation product by polyacrylamide gel electrophoresis is carried out by mixing samples from ligation reactions with Ficoll loading buffer and subjecting the mixture to electrophoresis in TBE (89 mM Tris, 89 mM Borate, 2 mM EDTA) in 20% polyacrylamide (Bio-Rad) urea (7M) gel at 600 V. for 1.5 hours. The gel is then wrapped in Saran Wrap and autoradiographed between two Lightning Plus intensifier screens (DuPont) overnight. Quantitation of ligation product is obtained either from densitometric measurements of the autoradiograph or directly from the gel via an AMBIS Radioisotope Scanning System II (Automated Microbiology System, Inc.). In the latter instance the polyacrylamide gel is first fixed in a 5% acetic acid solution for 10 minutes.

The ON-1⁻ON-2 ligation product can also be detected by hybridizing the product containing solution to a substrate containing an immobilized complementary DNA or RNA sequence under conditions whereby the ON-1⁻ON-2 ligation product will hybridize to the complementary sequence, but neither ON-1 nor ON-2 will. Bound label indicates presence of ligation product. The ligated product can also be detected in a subsequent round of ligation employing a radiolabeled counterpart substrate set (ON-3/ON-4). In the case where Ligated ON-3/ON-4 is to be detected, labeled ON-1/ON-2 would be used.

The product level after N-number of rounds of linear amplification-ligation is equal to N x Q where Q is the efficiency of the ligation. Q ranges from 0 to 1, where 1 is equivalent to 100% ligation.

### Example 2

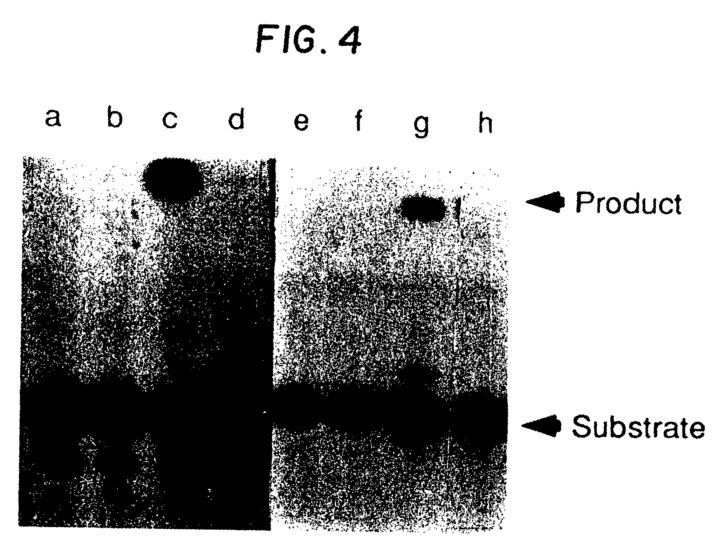

ON-1/ON-s2* and ON-s3/ON-4* were ligated linearly on oligonucleotide templates using the above methodology. ON-s2* and ON-s4* are the same as ON-s2 and ON-4 except that they are only 6 nucleotides long. 10 pmoles of ³²P-5' phosphorylated ON-s2* and unlabeled 5' OH-ON-1 (lanes a-d) or ³²P-5' phosphorylated ON-4* and unlabeled 5' OH-ON-s3 (lanes e-h) were ligated on 19 base long or 23 base long oligonucleotide template respectively at 30°C for 30 minutes with 1 U. of T4 DNA ligase in 10 µl reaction volume. Fig. 4 shows the results of the ligation amplification reactions described below. Samples in lanes a and e not treated with enzyme and samples in lanes b and f, without template, serve as negative controls. 1 pmole of the following oligonucleotide template was used: Hβ19S (c); Hβ19A (d); Hβ23S' (g); and Hβ23A' - (h).

As seen in Fig. 4, the expected ligation involving sickle cell template and sickle cell substrates occurred (lanes c and g); ligation of normal (A) beta globin substrates did not occur on the sickle cell template (lanes d and h).

### Example 3

The method of the present invention can also be used to exponentially amplify nucleic acid sequences. The major difference between exponential amplification ligation and the above-described linear amplification ligation is that all four oligonucleotides (ON-1/ON-2, and ON-3/ON-4) are present as substrates during each reaction round. The ligation product of either oligonucleotide set serves as the template for the other oligonucleotide set in subsequent amplification rounds as shown in Fig. 3.

Radioactively labeled substrate for detection can be included in the starting materials or can be added in the last round.

As is also the case for linear amplification, an estimation of the number of amplification rounds needed to produce a detectable signal must be made before carrying out the exponential amplification. After N rounds, the product formation equals (1 + Q)^{N}-1 where Q is ligation efficiency. The following table indicates the relative amounts of ligation products present after N cycles, assuming 100% efficiency at each cycle.

| Round | Fold Amplification (2)ⁿ-1 |
|---|---|
| 0 | 0 |
| 1 | 1 |
| 2 | 3 |
| 3 | 7 |
| 4 | 15 |
| 5 | 31 |
| 6 | 63 |
| 7 | 127 |
| 8 | 255 |
| 9 | 511 |
| 10 | 1023 |
| . | . |
| . | . |
| . | . |
| . | . |
| 15 | 32,767 |
| . | . |
| . | . |
| . | . |
| . | . |
| 20 | 1,048,575 |
| 21 | 2,097,141 |
| . | . |
| . | . |
| . | . |
| 25 | 33,554,431 |

For example, if 10⁶-fold of product amplification is required at a certain specific radioactivity, approximately 20 rounds of amplification are needed (2²⁰≅10⁶), assuming 100% ligation efficiency.

Near perfect ligation efficiency is present at later rounds where the primary template source is ligated oligonucleotide products. During early rounds, ligation efficiency may be lower depending upon reaction conditions, types of ligase used, and source of template.

Each round of exponential ligation is started by adding one unit of enzyme to the reaction mixture on ice. In the first and each subsequent round of amplification, the reaction mixture with the added enzyme is incubated for about 30 minutes at 30°C or for a longer period of time if template concentration is low. At the end of each round, the reaction mixture is quickly brought to boiling temperature for 5 minutes to inactivate the enzyme and dissociate the product strands from the template strands. Then the reaction mixture is centrifuged for 10 seconds and immediately cooled to about 0°C.

Another unit of T4 ligase is then added to initiate the second round of amplification. The mixture is then incubated, boiled, and cooled as above. At this point there are 3 copies of template present for each copy present at the beginning of the reaction. All subsequent rounds of amplification are carried out by the same sequence of steps for as many times needed in order to produce a detectable signal.

The progress of the amplification may be monitored by withdrawing from the reaction mixture an approximately 10 µl aliquot of reaction mixture during predetermined rounds. The withdrawn material is heat inactivated and is then combined with 2 to 3 µl of loading buffer. 2 µl of this buffered mixture is then loaded onto a 20% polyacrylamide gel and the gel is electrophoresed for one hour at 600V and then autoradiographed overnight with two DuPont Lightening-Plus intensifier screens.

Using these methods and the reaction mixture described in Example 1, 6.67 nM of oligonucleotide template, Hβ23S' and Hβ23A', was amplified with T4 DNA ligase and 2.33 µM of each flanking oligonucleotide substrate: ³²P-5' labeled ON-s2 (10⁶ cpm); ³²P-5' labeled ON-4 (10⁶ cpm); 5' OH-ON-1; and 5' OH-ON-s3. 1 U of T4 DNA ligase was used to initiate each round. Six rounds of ligation amplification were carried out.

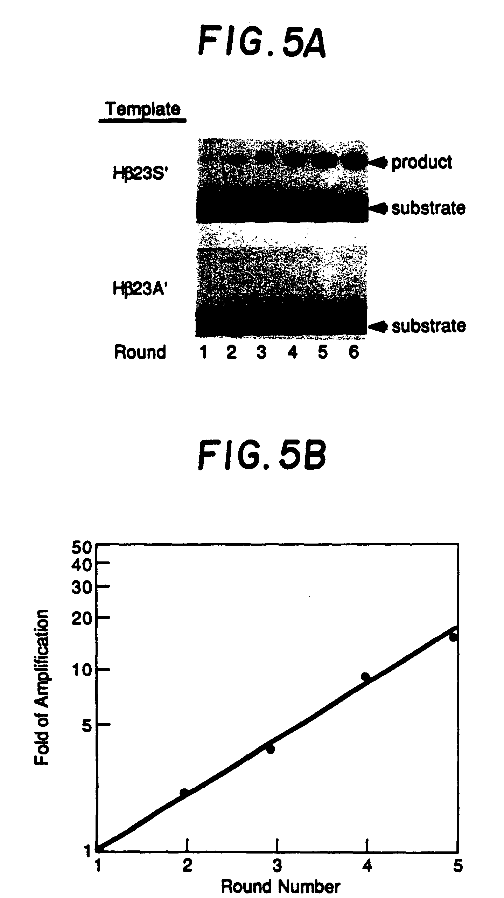

Fig. 5A is an autoradiogram showing the results of monitoring each round of reaction for each template. In rounds 2-6, the ligation reaction was specific for the Hβ23S' template containing the sickle cell sequence. The oligonucleotide substrates were not able to ligate in the presence of the Hβ23A' template.

Products of the exponential ligation method approximately double with each round. Fig. 5B shows a logarithmic plot of amplification folds as a function of the number of rounds of ligation amplification performed. A straight line was obtained with a calculated efficiency of 0.98. Thus, in each round, the ligation product of the preceding round serves effectively as the template for further oligonucleotide ligation.

The methods of the present invention can also be used to detect plasmid and genomic DNA sequences as illustrated in the following example.

### Example 4

Templates for amplification ligation were derived from plasmids pHβ^{a} and pHβ^{s} which contain respectively an approximately 4.4kbp normal (β^{a}) and sickle cell (β^{s}) beta globin gene insert. All DNA preparations were performed according to a modified Triton® X-100 procedure followed by Proteinase K and RNase treatment.

The plasmid DNA was treated with restriction enzyme (Bam HI and/or Taq I) and Exonuclease III (Exo III) prior to serving as template in ligation reactions as follows: pHβ^{a} and pHβ^{b} were separately digested with Bam HI (5 U/pmol) for 2 hours at 37°C and terminated in a boiling water bath for 5 minutes. Exo III (100 U/pmol) was then added to the reaction mixture with DTT to make a final concentration of 1 mM. The mixture was incubated 4 hours at 37°C. Phenol-chloroform extraction was performed for final purification.

1 nM of each of the two resulting templates (pHβ^{s} and pHβ^{a}) was then amplified exponentially using T4 DNA ligase and the reaction mixtures and methods described in Examples 1 and 3 except that the total volume of each reaction was 100 µl. 500 nM of each of the following oligonucleotide substrates was used: ³²P-5' labeled ON-s2 (10⁶ cpm), ³²P-5' labeled ON-4 (10⁶ cpm), 5' OH-ON-1, and 5' OH-ON-s3. After the 10th, 12th, and 14th amplification rounds, a 4 µl aliquot was withdrawn from the reaction mixture and analyzed by electrophoresis and autoradiography.

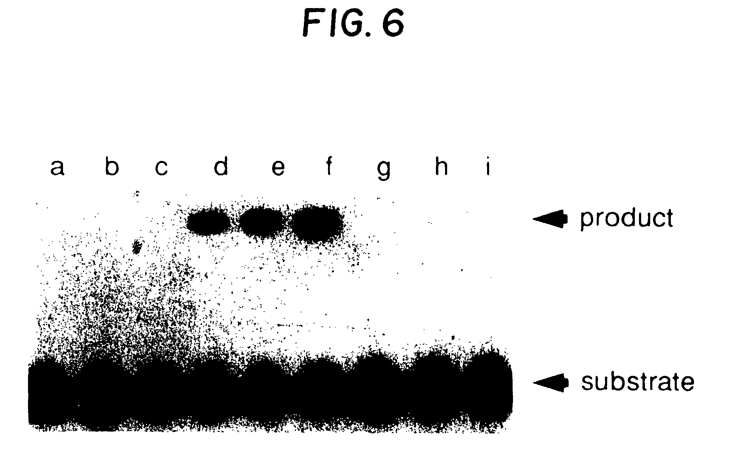

Fig. 6 shows the results of the ligation amplification. Product in lanes a-c resulted from reactions not including template; product in lanes d-f resulted from reactions containing pHβ^{s} template; and product in lanes g-i resulted from reactions containing pHβ^{a} template. Reaction products in lanes a, d, and g were withdrawn and analyzed after 10 rounds; products in lanes b, e, and h were analyzed after 12 rounds; and products in lanes c, f, and i were analyzed after 14 rounds.

No detectable ligation product is seen from the reactions involving pHβ^{a} which contained the normal beta globin sequence (g-i) or in lanes without any template (a-c). The pHβ^{s} sickle cell template was significantly amplified, with the detectable signal noticeably increasing between 10 (lane d), 12 (lane e), and 14 (lane f) amplification rounds.

For the 1 nM plasmid template used in the reactions, approximately 10 rounds of ligation amplification were necessary to generate a detectable signal corresponding to approximately a 50-fold amplification. In contrast, only about 3-6 rounds of amplification are needed to amplify oligonucleotide template of the same concentration to obtain the same fold amplification.

### Example 5

Normal (β^{a}/β^{a}), sickle cell disease (β^{s}/β^{s}), and sickle cell trait (β^{a}/β^{s}) genomic DNA samples were isolated from blood specimens of appropriate donors. Beta thalassemia major DNA was prepared from EBV transformed lymphocytes in culture (GM 2267 cells from NIGMS Human Genetic Mutant Cell Repository, Camden, N.J.). Thalassemia DNA was subsequently isolated from cultured cells. All DNA preparations were performed according to a modified Triton® X-100 procedure followed by Proteinase K and RNAse treatment.

Genomic DNA (5 µg) was digested with Taq I restriction enzyme (10 U/µg) (Boehringer Mannheim) overnight at 65°C and followed by Bam HI digestion (10 U./µg) (Bethesda Research Laboratory) for 8 hours at 37°C. Subsequently Exo III nuclease (100 U./pmol) was added to the reaction mixture along with DTT to make 1 mM final concentration and the reaction was incubated for 5 hours at 37°C. Phenol-chloroform extraction was carried out for final purification.

Ligation conditions for genomic DNA and for plasmid DNA are identical except that the total volume of the reaction for the genomic DNA sample is 200 µl.

Genomic DNA from β thalassemia, sickle cell, and normal DNA were amplified using 200 nM of the following sets of oligonucleotides: ³²P-5' labeled ON-s2 (10⁶ cpm)/³²P-5' labeled ON-4 (10⁶ cpm)/5' OH-ON-1/5' OH-ON-s3; and ³²P-5' labeled ON-2 (10⁶ cpm)/³²P-5' labeled ON-4 (10⁶ cpm)/5' OH-ON-1/5' OH-ON-3.

5 µg of genomic DNA contains approximately 500,000 molecules of homozygous single copy gene. When used as a template in a 200 µl reaction mixture, 5 ug of DNA is equivalent to 2.5 x 10⁻¹⁴M with respect to template. This concentration is well below the apparent Kₘ of T4 DNA ligase for ligation template. The enzyme activity is therefore expected to be slowed considerably. To overcome this kinetic constraint, early rounds of the ligation were incubated for a longer time period. The ligation time for initial rounds was 5 hours. With subsequent rounds, the incubation time was gradually reduced to 30 minutes. (Rounds 1-5 = 5 hours; round 6-10 = 4 hours; rounds 11-15 = 3 hours; rounds 16-20 = 2 hours; round 20-30 = 1 hour; and rounds 30 + = 30 minutes.) Each round however was not allowed to exceed 5 hours in order to avoid accumulation of blunt end products.

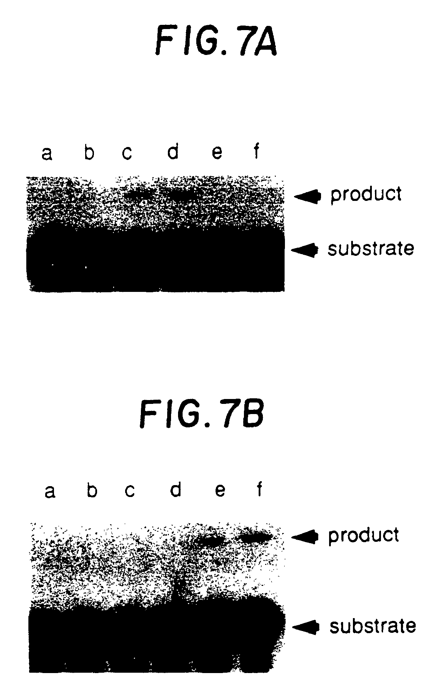

Figs. 7A and 7B show the results of 70-75 rounds of exponential amplification using these templates and oligonucleotides Fig. 7A contains amplification product resulting from the β^{s} oligonucleotide set; Fig. 7B contains amplification product resulting from the β^{a} oligonucleotide set. Lanes a and b of each autoradiogram contain reaction product using β thalassemia genomic DNA template; lanes c and d contain product from sickle cell genomic DNA template, and lanes e and f contain product using normal beta globin genomic DNA template. Template in lanes a, c, and e was amplified 70 rounds; template in lanes b, d, and f was amplified 75 rounds. The expected product bands appear in lanes c and d of Fig. 7A (sickle cell template and oligonucleotides) and in lanes e and f of Fig. 7B (normal beta globin template and oligonucleotides). β-thalassemia DNA (lanes a and b) and mismatched genomic DNA and substrates both showed absence of signal.

This example thus demonstrates that the ligation amplification methods of the present invention can be used as a diagnostic methodology on genomic DNA samples for sickle cell anemia.

### Example 6

5 µg of β-thalassemia sickle cell, and normal genomic DNA was each digested with Barn HI/Taq I and Exo III nuclease as described in Examples 4 and 5. Linear amplification was performed as described in Example 1. Oligonucleotides used were 100 fmoles of ³²P-5' labeled ON-s2 used directly from T4 kinase labeling reaction, without further purification, with DE-52 chromatography, and 100 fmoles of ON-1. The oligonucleotides were separately combined with the three templates in the presence of 200 mM NaCl and 0.5 U of T4 DNA ligase in total reaction volumes of 10 µl. The reaction mixture was incubated 3 hours at 30°C and the reaction was terminated by boiling. A second round of amplification was initiated by adding another 0.5 U. of enzyme. The reaction mixture was again incubated 3 hours. Three rounds of ligation amplification were performed. The resulting products were then electrophoresed and autoradiographed as described above.

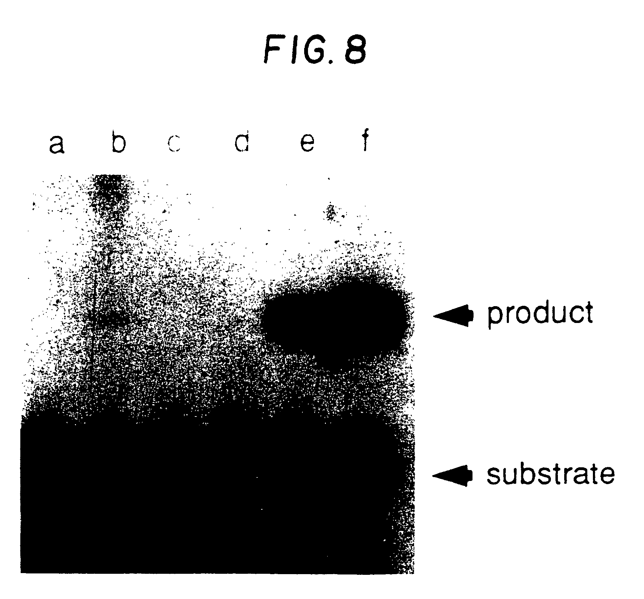

Fig. 8 shows the resulting autoradiogram. Lane a contains reaction product using β-thalassemia template, lane b contains reaction product using sickle cell template, and lane c contains reaction product using normal beta globin template. Positive controls were performed with 0.01, 1, and 10 fmoles of Hβ19S template (lanes d-f respectively). Lane b shows the expected product, whereas lanes a and c show an expected lack of product.

The methods of the present invention can also be used as a means to detect amplification product from other nucleic acid amplification methods such as the polymerase chain reaction.

### Example 7

Amplification reactions were performed with Polymerase Chain Reaction Kit (Gene Amp Kit, Perkin-Elmer Cetus) including 2.5 U of Thermus aquaticus DNA polymerase and 2.5 µM of oligonucleotide primers BGP1 and BGP2, 19 base long synthetic oligodeoxyribonucleotides which anneal to the beta globin gene at positions 256 nucleotides apart. The enriched 294 base pair fragment contains the β globin sequence of interest. 2 µg of the following genomic DNA were used as templates after being treated as described above: β thalassemia, homozygous sickle cell (β^{s}/β^{s}), normal β globin (β^{a}/β^{a}), and heterozygous sickle cell trait (β^{a}/β^{s}). 30 rounds of amplification gave approximately a 5 x 10⁵-fold amplification of the target sequence. 10 µl aliquots of the PCR enriched genomic DNA sequences were electrophoresed in 1.5% agarose gel at 60 V for 5 hours.

The electrophoretically separated products were then transferred to Genetran nylon membranes with 20X SSC (1X SSC = 150 mM NaCl and 15 mM Na citrate) according to the method of Southern, E.M., J. Mol. Biol. 98:503-517 (1975).

The Genetran membranes were directly hybridized to ³²P-5' labeled oligonucleotide probes HB19S or HB19A, in 5X SSPE (1X SSPE = 10 mM sodium phosphate pH 7.0, 0.18 M NaCl, and 1 mM EDTA), 1%-NaDodSO₄, 10 µg/ml of Homomix RNA, and 10⁶ cpm/ml of labeled oligonucleotide with 10 fold excess unlabelled competitor at 47°C for 2 hours. The membrane was first washed in room temperature with 6X SSC three times for 30 minutes. Subsequent wash in TMACI solution for 1 hour at 59°C removed all mismatch hybridization. (TMACI = 50 mM Tris, pH 8.0, 3 M tetramethylammonium chloride, 2 mM EDTA, 0.25% SDS).

Exponential ligation amplification was then performed on 20 µl samples of the PCR enriched sequences for 4 rounds with either 200 nM of β^{s} oligonucleotides (ON-1/ON-s2 and ON-s3/ON-4) or 200 nM of β^{a} oligonucleotides (ON-1/ON-a2 and ON-a3/ON-4) in a 100 µl or total reaction volume using T4 DNA ligase. 50 µl from each samples was analyzed by 20% polyacrylamide gel electrophoresis.

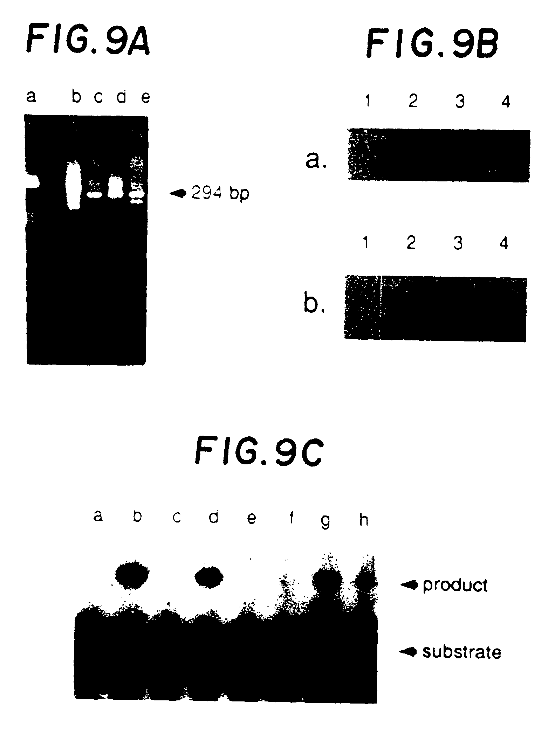

Ethidium bromide staining of the 1.5% agarose gel shows a single 294 base pair band (Fig.9A, lanes c-e). Lanes of Fig. 9A are: (a)-controi; (b)⁻β thalassemia; (c)⁻homozygous sickle cell trait; (d)⁻normal β globin; and (e)⁻heterozygous sickle cell trait. PCR amplification of thalassemia DNA (Panel A lane b) amplifies some nonspecific DNA's but shows no 294 base pair band.

Oligonucleotide hybridization analysis of the PCR enriched samples (samples from lanes b-e, Fig. 9A, correspond to lanes 1-4, Fig. 9B) immobilized on Genetran nylon membrane filter confirms that the enriched DNA's indeed contain beta globin sequence of interest (Fig. 9B). Competition hybridization of the filter as described in Nozari, G., et al., Gene 43:23 (1986) with either radioactively labelled Hβ19S and unlabelled Hβ19A (Fig. 9B, panel a) or labelled Hβ19A and unlabelled Hβ19S (Fig. 9B, panel b) correctly identifies sickle cell disease (β^{s}/β^{s}, Fig. 9B, lane 2, panel a), normal (β^{a}/β^{a}, Fig. 9B, lane 3, panel 2), and sickle cell trait (β^{a}/β^{s}, Fig. 9B, lane 4, panels a and b) DNA's.

The PCR enriched 256 base pair fragments ideally serve as template in the ligation analysis since they are relatively abundant which overcomes the ligase kinetic constraints (i.e., high Km for template). Ligation amplification performed on these 294 base pair templates (Fig. 9C templates: a,e ⁻β thalassemia; b,f ⁻Homozygous sickle cell disease; c,g⁻normal β globin; d,h -heterozygous sickle cell trait) with β^{s} (Fig. 9C, lanes a-d) oligonucleotide substrates showed positive ligation for sickle cell disease and sickle cell trait samples (Fig. 9C, lanes b and d). Ligation amplification with β^{a} (Fig. 9C, lanes e-h) oligonucleotide substrates is likewise positive for normal and sickle cell trait samples (Fig. 9C, lanes g and h).

This example demonstrates that ligation amplification can be used as a detection methodology coupled to other sequence amplification techniques.

## Claims

1. A method for amplifying a nucleic acid comprising a target sequence comprising the steps of:
(a) generating a single-stranded template comprising the target sequence;
(b) hybridising a pair of oligonucleotides to contiguous sequences of the template, said contiguous sequences comprising the target sequence, such that the oligonucleotides are juxtaposed for ligation on the template;
(c) ligating the juxtaposed oligonucleotides to produce a double-stranded ligation product;
(d) denaturing the double-stranded ligation product to produce an initial template strand and an oligonucleotide ligation product strand; and
(e) repeating steps (b) to (d) such that both the initial template strand and the oligonucleotide ligation strand act as templates for a further ligation reaction;
wherein steps (b) to (e) are conducted in a reaction mixture which contains at least 200 mM NaCl.

2. A method for amplifying a nucleic acid comprising a target sequence, said method comprising the steps of:
(a) generating a single-stranded template comprising the target sequence;
(b) amplifying said template by the polymerase chain reaction (PCR) to produce a PCR amplification product comprising multiple copies of said template comprising the target sequence;
(c) hybridising pairs of oligonucleotides to contiguous sequences of said copies of said template, said contiguous sequences comprising the target sequence, such that the oligonucleotides are juxtaposed for ligation on said template copies;
(d) ligating the juxtaposed oligonucleotides to produce double-stranded products;
(e) denaturing the double-stranded products to produce a plurality of initial template strands and a plurality of oligonucleotide product strands; and
(f) repeating steps (c) to (e) such that both the initial template strands and the oligonucleotide ligation strands act as templates for a further ligation reaction.

3. A method for simultaneously amplifying a plurality of nucleic acid sequences present in one or more nucleic acid molecules each comprising a target sequence, said method comprising the steps of:
(a) generating as appropriate a single-stranded template comprising the target sequences or a plurality of single-stranded templates each comprising a target sequence;
(b) hybridising a plurality of pairs of oligonucleotides to contiguous sequences of said template(s), said contiguous sequences comprising the target sequences, such that each of said oligonucleotide pairs is juxtaposed for ligation on a template;
(c) ligating the so juxtaposed oligonucleotide pairs to produce double-stranded ligation product.;
(d) denaturing double-stranded ligation product to produce initial template strands and oligonucleotide ligation product strands, and
(e) repeating steps (b) to (d) such that both initial template strands and oligonucleotide ligation product strands act as templates for further ligation reactions.

4. A method for the diagnosis of a genetic disease comprising analysing the genotype of a patient by a method which comprises amplifying a nucleic acid comprising a target sequence, said amplification method comprising the steps of :
(a) generating a single-stranded template comprising the target sequence;
(b) hybridising a pair of oligonucleotides to contiguous sequences of the template, said contiguous sequences comprising the target sequence, such that the oligonucleotides are juxtaposed for ligation on the template;
(c) ligating the juxtaposed oligonucleotides to produce a double-stranded ligation product;
(d) denaturing the double-stranded ligation product to produce an initial template strand and an oligonucleotide ligation product strand; and
(e) repeating steps (b) to (d) such that both the initial template strand and the oligonucleotide ligation product strand act as templates for a further ligation reaction.

5. A method according to any of claims 1 to 4 wherein the nucleic acid is DNA.

6. A method according to any of claims 1 to 4 wherein the nucleic acid is RNA.

7. A method for amplifying a nucleic acid comprising a target sequence from genomic DNA, said amplification method comprising the steps of:
(a) generating a single-stranded template comprising the target sequence;
(b) hybridising a pair of oligonucleotides to contiguous sequences of the template, said contiguous sequences comprising the target sequence, such that the oligonucleotides are juxtaposed for ligation on the template;
(c) ligating the juxtaposed oligonucleotides to produce a double-stranded ligation product;
(d) denaturing the double-stranded ligation product to produce an initial template strand and an oligonucleotide ligation product strand; and
(e) repeating steps (b) to (d) such that both the initial template strand and the oligonucleotide ligation product strand act as templates for a further ligation reaction.

8. A method for the diagnosis of a genetic disease comprising analysing the genotype of a patient by the method of any one of claims 1 to 7.

9. A method according to claim 7 or 8 wherein the disease is sickle cell anaemia.

10. A method according to any one of claims 1 to 9 wherein single-stranded template is generated by disassociating the complementary strands of a double-stranded nucleic acid.

11. A method according to claim 10 wherein both complementary strands are used as templates.

12. A method according to any one of claims 1 to 11 further comprising producing an amplification product by repetition of step (e) and subsequently determining the presence or absence of a nucleic acid sequence complementary to or identical to the target sequence in the amplification product.

13. A method for amplifying a nucleic acid comprising a target sequence comprising the steps of:
(a) generating a single-stranded template comprising the target sequence;
(b) hybridising a pair of oligonucleotides to contiguous sequences of the template, said contiguous sequences comprising the target sequence, such that the oligonucleotides are juxtaposed for ligation on the template;
(c) ligating the juxtaposed oligonucleotides to produce a double-stranded ligation product;
(d) denaturing the double-stranded ligation product to produce an initial template strand and an oligonucleotide ligation product strand; and
(e) repeating steps (b) to (d) such that both the initial template strand and the oligonucleotide ligation strand act as templates for a further ligation reaction;
wherein the template nucleic acid is from bacteria.

14. A method for amplifying a nucleic acid comprising a target sequence comprising the steps of:
(a) generating a single-stranded template comprising the target sequence;
(b) hybridising a pair of oligonucleotides to contiguous sequences of the template, said contiguous sequences comprising the target sequence, such that the oligonucleotides are juxtaposed for ligation on the template;
(c) ligating the juxtaposed oligonucleotides to produce a double-stranded ligation product;
(d) denaturing the double-stranded ligation product to produce an initial template strand and an oligonucleotide ligation product strand; and
(e) repeating steps (b) to (d) such that both the initial template strand and the oligonucleotide ligation strand act as templates for a further ligation reaction;
wherein the template nucleic acid is from yeast.

15. A method for amplifying a nucleic acid comprising a target sequence comprising the steps of:
(a) generating a single-stranded template comprising the target sequence;
(b) hybridising a pair of oligonucleotides to contiguous sequences of the template, said contiguous sequences comprising the target sequence, such that the oligonucleotides are juxtaposed for ligation on the template;
(c) ligating the juxtaposed oligonucleotides to produce a double-stranded ligation product;
(d) denaturing the double-stranded ligation product to produce an initial template strand and an oligonucleotide ligation product strand; and
(e) repeating steps (b) to (d) such that both the initial template strand and the oligonucleotide ligation strand act as templates for a further ligation reaction;
wherein the template nucleic acid is from a virus.

16. A method according to any of claims 13 to 15 wherein the nucleic acid is DNA.

17. A method according to any of claims 13 to 15 wherein the nucleic acid is RNA.

## Patentansprüche

1. Verfahren zur Amplifikation einer Nucleinsäure mit einer Zielsequenz, umfassend die Schritte:
(a) Erzeugung einer einzelsträngigen Matrize mit der Zielsequenz,
(b) Hybridisierung eines Oligonucleotidpaares an angrenzende Sequenzen der Matrize, wobei die angrenzenden Sequenzen die Zielsequenz umfassen, so daß die Oligonucleotide an die Matrize zur Ligation aneinander gelagert werden,
(c) Ligation der aneinander gelagerten Oligonucleotide, um ein doppelsträngiges Ligationsprodukt herzustellen,
(d) Denaturierung des doppelsträngigen Ligationsproduktes, um einen ursprünglichen Matrizenstrang und einen Oligonucleotid-Ligationsproduktstrang herzustellen, und
(e) Wiederholung der Schritte (b) bis (d), so daß beide, der ursprüngliche Matrizenstrang und der Oligonucleotid-Ligationsstrang, als Matrizen für eine weitere Ligationsreaktion fungieren,
wobei die Schritte (b) bis (e) in einem Reaktionsgemisch, welche mindestens 200 mmol NaCl enthält, durchgeführt werden.

2. Verfahren zur Amplifikation einer Nucleinsäure mit einer Zielsequenz, wobei das Verfahren die Schritte umfaßt:
(a) Erzeugung einer einzelsträngigen Matrize mit der Zielsequenz,
(b) Amplifikation der Matrize durch Polymerasekettenreaktion (PCR), um ein PCR-Amplifikationsprodukt herzustellen, welches vielfache Kopien der Matrize mit der Zielsequenz umfaßt,
(c) Hybridisierung von Oligonucleotidpaaren an angrenzende Sequenzen der Kopien der Matrize, wobei die angrenzenden Sequenzen die Zielsequenz umfassen, so daß die Oligonucleotide an die Matrizenkopien zur Ligation aneinander gelagert werden,
(d) Ligation der aneinander gelagerten Oligonucleotide, um doppelsträngige Produkte herzustellen,
(e) Denaturierung des doppelsträngigen Produktes, um eine Vielzahl der ursprünglichen Matrizenstränge und eine Vielzahl der Oligonucleotid-Produktstränge herzustellen, und
(f) Wiederholung der Schritte (c) bis (e), so daß beide, die ursprünglichen Matrizenstränge und die Oligonucleotid-Ligationsstränge, als Matrizen für eine weitere Ligationsreaktion fungieren.

3. Verfahren zur gleichzeitigen Amplifikation einer Vielzahl von Nucleinsäuresequenzen, die in einem oder mehreren Nucleinsäuremolekülen vorliegen, welche jede eine Zielsequenz umfassen, wobei das Verfahren die Schritte umfaßt:
(a) Erzeugung, wie angemessen, einer einzelsträngigen Matrize mit Zielsequenzen oder einer Vielzahl von einzelsträngigen Matrizen, welche jede eine Zielsequenz umfassen,
(b) Hybridisierung einer Vielzahl von Oligonucleotidpaaren an angrenzende Sequenzen der Matrize(n), wobei die angrenzenden Sequenzen die Zielsequenzen umfassen, so daß jedes der Oligonucleotidpaare an eine Matrize zur Ligation aneinander gelagert wird,
(c) Ligation der so aneinander gelagerten Oligonucleotidpaare, um ein doppelsträngiges Ligationsprodukt herzustellen,
(d) Denaturierung des doppelsträngigen Ligationsprodukts, um die ursprünglichen Matrizenstränge und Oligonucleotid-Ligationsproduktstränge herzustellen, und
(e) Wiederholung der Schritte (b) bis (d), so daß beide, die ursprünglichen Matrizenstränge und die Oligonucleotid-Ligationsproduktstränge, als Matrizen für weitere Ligationsreaktionen fungieren.

4. Verfahren zur Diagnose einer Erbkrankheit, welches das Analysieren des Genotyps eines Patienten durch ein Verfahren umfaßt, welches die Amplifikation einer Nucleinsäure umfaßt, die eine Zielsequenz umfaßt, wobei das Amplifikationsverfahren die Schritte umfaßt:
(a) Erzeugung einer einzelsträngigen Matrize einer Zielsequenz,
(b) Hybridisierung eines Oligonucleotidpaares an angrenzende Sequenzen der Matrize, wobei die angrenzenden Sequenzen die Zielsequenz umfassen, so daß die Oligonucleotide an die Matrize zur Ligation aneinander gelagert werden,
(c) Ligation der aneinander gelagerten Oligonucleotide, um ein doppelsträngiges Ligationsprodukt herzustellen,
(d) Denaturierung des doppelsträngigen Ligationsproduktes, um einen ursprünglichen Matrizenstrang und einen Oligonucleotid-Ligationsproduktstrang herzustellen, und
(e) Wiederholung der Schritte (b) bis (d), so daß beide, der ursprüngliche Matrizenstrang und der Oligonucleotid-Ligationsproduktstrang, als Matrizen für eine weiter Ligationsreaktion fungieren.

5. Verfahren nach einem der Ansprüche 1 bis 4, worin die Nucleinsäure DNA ist.

6. Verfahren nach einem der Ansprüche 1 bis 4, worin die Nucleinsäure RNA ist.

7. Verfahren zur Amplifikation einer Nucleinsäure mit einer Zielsequenz aus genomischer DNA, wobei das Amplifikationsverfahren die Schritte umfaßt:
(a) Erzeugung einer einzelsträngigen Matrize mit der Zielsequenz,
(b) Hybridisierung eines Oligonucleotidpaares an angrenzende Sequenzen der Matrize, wobei die angrenzenden Sequenzen die Zielsequenz umfassen, so daß die Oligonucleotide an die Matrize zur Ligation aneinander gelagert werden,
(c) Ligation der aneinander gelagerten Oligonucleotide, um ein zweisträngiges Ligationsprodukt herzustellen,
(d) Denaturierung des doppelsträngigen Ligationsprodukts, um einen ursprünglichen Matrizenstrang und einen Oligonucleotid-Ligationsproduktstrang herzustellen, und
(e) Wiederholung der Schritte (b) bis (d), so daß beide, der ursprüngliche Matrizenstrang und der Oligonucleotid-Ligationsproduktstrang, als Matrizen für eine weitere Ligationsreaktion fungieren.

8. Verfahren zur Diagnose von Erbkrankheiten, umfassend das Analysieren des Genotyps eines Patienten durch das Verfahren nach einem der Ansprüche 1 bis 7.

9. Verfahren nach Anspruch 7 oder 8, wobei die Erkrankung Sichelzellanämie ist.

10. Verfahren nach einem der Ansprüche 1 bis 9, wobei die einzelsträngige Matrize durch Dissoziierung der komplementären Stränge einer doppelsträngigen Nucleinsäure erzeugt wird.

11. Verfahren nach Anspruch 10, wobei beide komplementären Stränge als Matrizen verwendet werden.

12. Verfahren nach einem der Ansprüche 1 bis 11, ferner umfassend das Herstellen eines Amplifikationsproduktes durch Wiederholung des Schrittes (e) und anschließendem Bestimmen der Anwesenheit oder Abwesenheit einer Nucleinsäuresequenz, die komplementär oder identisch zu der Zielsequenz im Amplifikationsprodukt ist.

13. Verfahren zur Amplifikation einer Nucleinsäure mit einer Zielsequenz, umfassend die Schritte:
(a) Erzeugung einer einzelsträngigen Matrize mit der Zielsequenz,
(b) Hybridisierung eines Oligonucleotidpaares an angrenzende Sequenzen der Matrize, wobei die angrenzenden Sequenzen die Zielsequenz umfassen, so daß die Oligonucleotide an die Matrize zur Ligation aneinander gelagert werden,
(c) Ligation der aneinander gelagerten Oligonucleotide, um einen doppelsträngiges Ligationsprodukt herzustellen,
(d) Denaturierung des doppelsträngigen Ligationsprodukts, um einen ursprünglichen Matrizenstrang und einen Oligonucleotid-Ligationsproduktstrang herzustellen, und
(e) Wiederholung der Schritte (b) bis (d), so daß beide, der ursprüngliche Matrizenstrang und der Oligonucleotid-Ligationsstrang, als Matrizen für eine weitere Ligationsreaktion fungieren,
wobei die Matrizennucleinsäure aus Bakterien stammt.

14. Verfahren zur Amplifikation einer Nucleinsäure mit einer Zielsequenz, umfassend die Schritte:
(a) Erzeugung einer einzelsträngigen Matrize mit der Zielsequenz,
(b) Hybridisierung eines Oligonucleotidpaares an angrenzende Sequenzen der Matrize, wobei die angrenzenden Sequenzen die Zielsequenz umfassen, so daß die Oligonucleotide an die Matrize zur Ligation aneinander gelagert werden,
(c) Ligation der aneinander gelagerten Oligonucleotide, um ein doppelsträngiges Ligationsprodukt herzustellen,
(d) Denaturierung des doppelsträngigen Ligationsprodukts, um einen ursprünglichen Matrizenstrang und einen Oligonucleotid-Ligationsproduktstrang herzustellen, und
(e) Wiederholung der Schritte (b) bis (d), so daß beide, der ursprüngliche Matrizenstrang und der Oligonucleotid-Ligationsstrang als Matrizen für eine weitere Ligationsreaktion fungieren,
wobei die Matrizennucleinsäure aus Hefe stammt.

15. Verfahren zur Amplifikation einer Nucleinsäure mit einer Zielsequenz, umfassend die Schritte:
(a) Erzeugung einer einzelsträngigen Matrize mit der Zielsequenz,
(b) Hybridisierung eines Oligonucleotidpaares an angrenzende Sequenzen der Matrize, wobei die angrenzenden Sequenzen die Zielsequenz umfassen, so daß die Oligonucleotide an die Matrize zur Ligation aneinander gelagert werden, und
(c) Ligation der aneinander gelagerten Oligonucleotide, um ein doppelsträngiges Ligationsprodukt herzustellen,
(d) Denaturierung des doppelsträngigen Ligationsproduktes, um einen ursprünglichen Matrizenstrang und einen Oligonucleotid-Ligationsproduktstrang herzustellen, und
(e) Wiederholung der Schritte (b) bis (d), so daß beide, der ursprüngliche Matrizenstrang und der Oligonucleotid-Ligationsstrang, als Matrizen für eine weitere Ligationsreaktion fungieren, wobei die Matrizennucleinsäure aus einem Virus stammt.

16. Verfahren nach einem der Ansprüche 13 bis 15, worin die Nucleinsäure DNA ist.

17. Verfahren nach einem der Ansprüche 13 bis 15, worin die Nucleinsäure RNA ist.

## Revendications

1. Procédé pour amplifier un acide nucléique comprenant une séquence cible, qui comprend les étapes consistant :
(a) à produire une matrice simple brin comprenant une séquence cible ;
(b) à hybrider une paire d'oligonucléotides à des séquences contiguës de la matrice, lesdites séquences contiguës comprenant la séquence cible, de façon que les oligonucléotides soient juxtaposés pour la ligature à la matrice ;
(c) à ligaturer les oligonucléotides juxtaposés pour obtenir un produit de ligature double brin ;
(d) à dénaturer le produit de ligature double brin, pour obtenir un brin de matrice initial et un brin de produit de ligature des oligonucléotides ; et
(e) à répéter les étapes (b) à (d) de façon que les deux brins, de matrice initial et de ligature des oligonucléotides, jouent le rôle de matrices pour une réaction ultérieure de ligature ;
dans lequel les étapes (b) à (e) sont conduites dans un mélange réactionnel qui contient au moins 200mM de NaCl.

2. Procédé pour amplifier un acide nucléique comprenant une séquence cible, ledit procédé comprenant les étapes consistant :
(a) à produire une matrice simple brin comprenant une séquence cible ;
(b) à amplifier ladite matrice par l'amplification en chaîne par polymérase (PCR) pour obtenir un produit à amplification PCR comprenant des copies multiples de ladite matrice comprenant la séquence cible ;
(c) à hybrider une paire d'oligonucléotides à des séquences contiguës desdites copies de ladite matrice, lesdites séquences contiguës comprenant la séquence cible, de façon que les oligonucléotides soient juxtaposés pour la ligature sur lesdites copies de matrice ;
(d) à ligaturer les oligonucléotides juxtaposés pour obtenir des produits double brin ;
(e) à dénaturer les produits double brin, pour obtenir une pluralité de brins de matrice initial et une pluralité de brins de produits des oligonucléotides ; et
(f) à répéter les étapes (c) à (e) de façon que les deux brins, de matrice initial et de ligature des oligonucléotides, jouent le rôle de matrices pour une réaction ultérieure de ligature.

3. Procédé pour amplifier simultanément une pluralité de séquences d'acide nucléique présente dans une ou plus molécules d'acide nucléique comprenant chacune une séquence cible, ledit procédé comprenant les étapes consistant :
(a) à produire telle qu'appropriée une matrice simple brin comprenant les séquences cible ou une pluralité de matrices simple brin comprenant chacune une séquence cible ;
(b) à hybrider une pluralité de paires d'oligonucléotides à des séquences contiguës de ladite matrice(s), lesdites séquences contiguës comprenant les séquences cible, de façon que chacune desdites paires d'oligonucléotides soit juxtaposée pour la ligature à une matrice ;
(c) à ligaturer aussi les paires d'oligonucléotides juxtaposées pour obtenir un produit de ligature double brin ;
(d) à dénaturer le produit de ligature double brin, pour obtenir des brins de matrice initial et des brins de produit de ligature des oligonucléotides ; et
(e) à répéter les étapes (b) à (d) de façon que les deux brins, de matrice initial et de produit de ligature des oligonucléotides, jouent le rôle de matrices pour des réactions ultérieures de ligature.

4. Procédé pour le diagnostic d'une maladie génétique comprenant l'analyse du génotype d'un patient par le procédé qui consiste à amplifier l'acide nucléique comprenant une séquence cible, ledit procédé d'amplification comprenant les étapes consistant :
(a) à produire une matrice simple brin comprenant une séquence cible ;
(b) à hybrider une paire d'oligonucléotides à des séquences contiguës de la matrice, lesdites séquences contiguës comprenant la séquence cible, de façon que les oligonucléotides soient juxtaposés pour la ligature à la matrice ;
(c) à ligaturer les oligonucléotides juxtaposés pour obtenir un produit de ligature double brin ;
(d) à dénaturer le produit de ligature double brin, pour obtenir un brin de matrice initial et un brin de produit de ligature des oligonucléotides ; et
(e) à répéter les étapes (b) à (d) de façon que les deux brins, de matrice initial et de produit de ligature des oligonucléotides, jouent le rôle de matrices pour une réaction ultérieure de ligature.

5. Procédé selon l'une quelconque des revendications 1 à 4, dans lequel l'acide nucléique est un ADN.

6. Procédé selon l'une quelconque des revendications 1 à 4, dans lequel l'acide nucléique est un ARN.

7. Procédé pour amplifier un acide nucléique comprenant une séquence cible issue d'un ADN génomique, ledit procédé d'amplification comprend les étapes consistant :
(a) à produire une matrice simple brin comprenant une séquence cible ;
(b) à hybrider une paire d'oligonucléotides à des séquences contiguës de la matrice, lesdites séquences contiguës comprenant la séquence cible, de façon que les oligonucléotides soient juxtaposés pour la ligature à la matrice ;
(c) à ligaturer les oligonucléotides juxtaposés pour obtenir un produit de ligature double brin ;
(d) à dénaturer le produit de ligature double brin, pour obtenir un brin de matrice initial et un brin de produit de ligature des oligonucléotides ; et
(e) à répéter les étapes (b) à (d) de façon que les deux brins, de matrice initial et de ligature des oligonucléotides, jouent le rôle de matrices pour une réaction ultérieure de ligature.

8. Procédé pour le diagnostic d'une maladie génétique, qui consiste à analyser le génotype d'un patient par le procédé selon l'une quelconque des revendications 1 à 7.

9. Procédé selon la revendication 7 ou 8, dans lequel la maladie est la drépanocytose.

10. Procédé selon l'une quelconque des revendications 1 à 9, dans lequel la matrice simple brin est produite par dissociation des brins complémentaires d'un acide nucléique double brin.

11. Procédé selon la revendication 10, dans lequel les deux brins complémentaires sont utilisés comme matrices.

12. Procédé selon l'une quelconque des revendications 1 à 11 qui consiste en outre à produire un produit d'amplification par répétition de l'étape (e), puis à déterminer la présence ou l'absence d'une séquence d'acide nucléique complémentaire de la séquence cible, ou identique à la séquence cible, dans le produit d'amplification.

13. Procédé pour amplifier un acide nucléique comprenant une séquence cible, qui comprend les étapes consistant :
(a) à produire une matrice simple brin comprenant une séquence cible ;
(b) à hybrider une paire d'oligonucléotides à des séquences contiguës de la matrice, lesdites séquences contiguës comprenant la séquence cible, de façon que les oligonucléotides soient juxtaposés pour la ligature à la matrice ;
(c) à ligaturer les oligonucléotides juxtaposés pour obtenir un produit de ligature double brin ;
(d) à dénaturer le produit de ligature double brin, pour obtenir un brin de matrice initial et un brin de produit de ligature des oligonucléotides ; et
(e) à répéter les étapes (b) à (d) de façon que les deux brins, de matrice initial et de ligature des oligonucléotides, jouent le rôle de matrices pour une réaction ultérieure de ligature ;
dans lequel la matrice de l'acide nucléique est issue d'une bactérie.

14. Procédé pour amplifier un acide nucléique comprenant une séquence cible, qui comprend les étapes consistant :
(a) à produire une matrice simple brin comprenant une séquence cible ;
(b) à hybrider une paire d'oligonucléotides à des séquences contiguës de la matrice, lesdites séquences contiguës comprenant la séquence cible, de façon que les oligonucléotides soient juxtaposés pour la ligature à la matrice ;
(c) à ligaturer les oligonucléotides juxtaposés pour obtenir un produit de ligature double brin ;
(d) à dénaturer le produit de ligature double brin, pour obtenir un brin de matrice initial et un brin de produit de ligature des oligonucléotides ; et
(e) à répéter les étapes (b) à (d) de façon que les deux brins, de matrice initial et de ligature des oligonucléotides, jouent le rôle de matrices pour une réaction ultérieure de ligature ;
dans lequel la matrice de l'acide nucléique est issue d'une levure.

15. Procédé pour amplifier un acide nucléique comprenant une séquence cible, qui comprend les étapes consistant :
(a) à produire une matrice simple brin comprenant une séquence cible ;
(b) à hybrider une paire d'oligonucléotides à des séquences contiguës de la matrice, lesdites séquences contiguës comprenant la séquence cible, de façon que les oligonucléotides soient juxtaposés pour la ligature à la matrice ;
(c) à ligaturer les oligonucléotides juxtaposés pour obtenir un produit de ligature double brin ;
(d) à dénaturer le produit de ligature double brin, pour obtenir un brin de matrice initial et un brin de produit de ligature des oligonucléotides ; et
(e) à répéter les étapes (b) à (d) de façon que les deux brins, de matrice initial et de ligature des oligonucléotides, jouent le rôle de matrices pour une réaction ultérieure de ligature ;
dans lequel la matrice de l'acide nucléique est issue d'un virus.

16. Procédé selon l'une quelconque des revendications 13 à 15, dans lequel l'acide nucléique est un ADN.

17. Procédé selon l'une quelconque des revendications 13 à 15, dans lequel l'acide nucléique est un ARN.
